(19) Europäisches Patentamt

European Patent Office

Office européen des brevets

(11) Veröffentlichungsnummer: **0 596 470 B1**

(12) **EUROPÄISCHE PATENTSCHRIFT**

(45) Veröffentlichungstag der Patentschrift: **09.08.95**

(21) Anmeldenummer: **93117808.1**

(22) Anmeldetag: **03.11.93**

(51) Int. Cl.⁶: **C07C 9/04**, C07C 7/00

(54) **Verfahren zur Gewinnung von hochreinem flüssigen Methan.**

(30) Priorität: **06.11.92 DE 4237620**

(43) Veröffentlichungstag der Anmeldung:
**11.05.94 Patentblatt 94/19**

(45) Bekanntmachung des Hinweises auf die
Patenterteilung:
**09.08.95 Patentblatt 95/32**

(84) Benannte Vertragsstaaten:
**AT BE CH DE DK FR LI NL SE**

(56) Entgegenhaltungen:
**US-A- 4 936 887**

(73) Patentinhaber: **Linde Aktiengesellschaft
Abraham-Lincoln-Strasse 21
D-65189 Wiesbaden (DE)**

(72) Erfinder: **Förg, Wolfgang, Dipl.-Phys.
Walchstadter Höhe 1
D-82057 Icking (DE)**
Erfinder: **Stockmann, Rudolf, Dipl.-Ing.
Westendstrasse 7
D-86807 Buchloe (DE)**

(74) Vertreter: **Kasseckert, Rainer
Linde Aktiengesellschaft,
Zentrale Patentabteilung
D-82049 Höllriegelskreuth (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Gewinnung von hochreinem flüssigen Methan aus einem im wesentlichen aus Methan, $C_{2+}$-Kohlenwasserstoffen, Kohlendioxid und Stickstoff bestehenden Einsatzgasgemisch.

Hochreines, flüssiges Methan wird in zunehmendem Maße als umweltfreundlicher Treibstoff für Dieselmotoren von Lokomotiven, Bussen und Lastkraftwagen verwendet. Das flüssige Methan wird dabei aus Erdgas gewonnen. Aus der US-Patentschrift Nr. 4 761 167 ist ein Verfahren zum Gewinnen von Methan aus einem dieses, $C_{2+}$-Kohlenwasserstoffe, Kohlendioxid und Stickstoff enthaltenden Gasgemisch bekannt. Hierbei wird das Einsatzgasgemisch zunächst einer kryogenen Destillation zugeführt, in der die $C_{2+}$-Kohlenwasserstoff- sowie eine erste Kohlendioxidfraktion gewonnen werden. Das verbleibende $CH_4/N_2/CO_2$-Gasgemisch wird anschließend in eine Druckwechseladsorption geleitet, und in dieser von Kohlendioxid befreit. In einer der Druckwechseladsorption nachgeschalteten Stickstoff-Abtrenneinheit erfolgt dann eine Zerlegung des Gasgemisches in die Methanprodukt- sowie in eine Stickstoff-Fraktion, wobei letzte-re zur Regenerierung der mit Kohlendioxid beladenen Adsorber verwendet wird. Dieser Prozeß findet vor allem dann Verwendung, wenn die Stickstoffkonzentration im Einsatzgasgemisch etwa in der gleichen Größenordnung wie die Methankonzentration liegt.

Eine zu diesem Prozeß alternative Verfahrensweise zeichnet sich dadurch aus, daß an Stelle der kryogenen Destillation eine Amin-Wäsche zur Kohlendioxid-Abtrennung aus dem Einsatzgasgemisch vorgesehen ist. In diesem Falle muß die anschließende Adsorptionsanlage zur Trocknung des nach der Amin-Wäsche wassergesättigten Einsatzgasgemisches verwendet werden.

Diese Verfahrensweise hat jedoch den Nachteil, daß die Adsorptionsanlage, aufgrund der vollständigen Wassersättigung des aus der Amin-Wasche austretenden Gasstromes, sehr groß gebaut werden muß. Ferner muß für eine Entsorgung bzw. Wiederverwendung des in der Kohlendioxid-Abtrennung benötigten Waschmittels Sorge getragen werden.

Ziel und Aufgabe der vorliegenden Erfindung ist es, ein Verfahren zur Gewinnung von hochreinem flüssigen Methan anzugeben, bei dem zum einen auf eine Amin-Wäsche verzichtet werden kann und zum anderen eine, im Vergleich mit den zum Stand der Technik zählenden Verfahren kleinere Adsorptionsanlage benötigt wird.

Dies wird erfindungsgemäß dadurch erreicht, daß

a) das Einsatzgasgemisch zunächst einer Adsorptionsanlage zugeführt und in dieser von Wasser befreit,

b) das getrocknete Einsatzgasgemisch einer Membrantrennanlage zugeführt und in dieser das Kohlendioxid bis auf einen Restgehalt kleiner als 2 Vol-% abgetrennt und

c) das nunmehr im wesentlichen aus Methan, $C_{2+}$-Kohlenwasserstoffen und Stickstoff bestehende Gasgemisch einer Tieftemperatur-Destillation zugeführt und in dieser die $C_{2+}$-Kohlenwasserstoffe sowie der Restgehalt an Kohlendioxid durch Destillation und der Stickstoff durch Entspannen abgetrennt werden, wodurch ein hochreiner flüssiger Methanproduktstrom gewonnen wird.

Die erfindungsgemäße Kombination aus Adsorption zur Trocknung des Einsatzgasstromes, Membrantrennanlage zur Kohlendioxidentfernung und Tieftemperatur-Destillation zur Gewinnung des hochreinen flüssigen Methanproduktstromes resultiert in einem sehr leicht zu betreibenden, schnell anzufahrenden und kostengünstigen Verfahren. Mittels der Adsorption kann neben im Einsatzgasgemisch enthaltenem Wasser auch Glykol entfernt werden. Adsorptionsverfahren zur Trocknung von Gasströmen aller Art sind aus der Literatur bekannt.

In der Membrantrennanlage läßt sich bei Wahl geeigneter Membranen eine Reduzierung des Kohlendioxid-Gehalts auf weniger als 2 Vol-% erreichen. Bei der in der anschließenden Tieftemperatur-Destillationstattfindenden Verflüssigung wird das restliche Kohlendioxid gemeinsam mit den $C_{2+}$-Kohlenwasserstoffen bis zu einer Konzentration von weniger als 50 ppm entfernt. Der Aufwand dafür, daß die Tieftemperatur-Destillation auch zur Abtrennung des noch im Gasstrom verbliebenen Kohlendioxids verwendet wird, ist gering. Dieser Mehraufwand wird auch bereits dadurch kompensiert, daß der aus der Membrantrennanlage austretende Gasstrom mit etwa 300 K in die Tieftemperatur-Destillation eintritt. Im Falle einer der Tieftemperatur-Destillation vorgeschalteten Amin-Wäsche läge diese Temperatur bei etwa 322 K. Dies wiederum würde einen Mehraufwand an Energie für die Verflüssigung innerhalb der Tieftemperatur-Destillation zur Folge haben.

Eine Ausgestaltung des erfindungsgemäßen Verfahrens ist dadurch gekennzeichnet, daß die in der Membrantrennanlage gewonnene Kohlendioxidfraktion zusammen mit der $C_{2+}$-Kohlenwasserstofffraktion aus der Tieftemperatur-Destillation und dem Boil-off-Gas aus einem diesem Verfahren nachgeschaltetem Flüssigmethan-Speichertank vermischt und der Adsorptionsanlage als Regeneriergas zugeführt wird.

EP 0 596 470 B1

Die Erfindung weiterbildend wird vorgeschlagen, die in der Membrantrennanlage gewonnene Kohlendioxidfraktion zusammen mit dem Boil-off-Gas vor der Vermischung mit der $C_{2+}$-Kohlenwasserstofffraktion ein- oder mehrstufig zu verdichten.

Eine weitere vorteilhafte Ausgestaltung des erfindungsgemäßen Verfahrens ist dadurch gekennzeichnet, daß in der Tieftemperatur-Destillation eine Kolonne zur Abtrennung der $C_{2+}$-Kohlenwasserstoffe und des restlichen Kohlendioxids verwendet wird, die mittels des im wesentlichen aus Methan, $C_{2+}$-Kohlenwasserstoffen und Stickstoff bestehenden Gasgemisches aus der Membrantrennanlage geheizt und durch einen Teilstrom des in der Tieftemperatur-Destillation verwendeten Kältekreislaufs gekühlt wird.

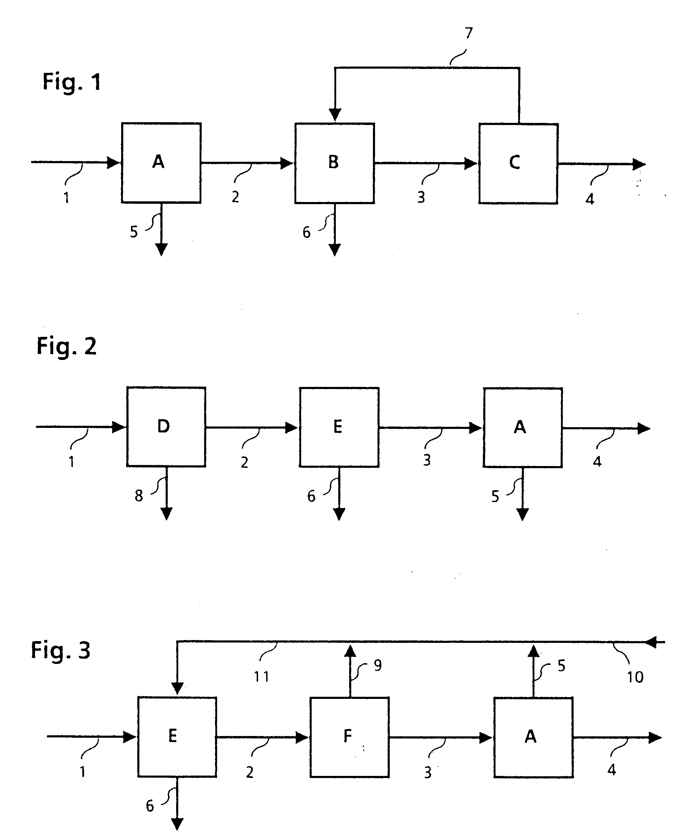

Die Erfindung sowie weitere Einzelheiten der Erfindung werden im folgenden anhand der Figuren 1 bis 5 näher erläutert. Hierbei besitzen identische Verfahrensteile gleiche Bezugszeichen.

**Figur 1** stellt einen Prozeß dar, wie er in dem US- Patent Nr. 4 761 167 beschrieben ist. Hierbei wird über Leitung 1 der Einsatzgasstrom in die kryogene Destillation A geführt und in dieser die $C_{2+}$-Kohlenwasserstoffe sowie ein erster Kohlendioxidstrom über Leitung 5 gewonnen. Über Leitung 2 wird der Druckwechseladsorption B ein $CH_4/N_2/CO_2$-Gasgemisch zugeführt. In ihr erfolgt eine adsorptive Auftrennung dieses Gasgemisches in eine $CO_2$-reiche Fraktion, die über Leitung 6 abgegeben, und eine $CH_4/N_2$-reiche Fraktion, die über Leitung 3 der Stickstoffabtrenneinheit C zugeführt wird. Während der Methanproduktstrom über Leitung 4 gewonnen wird, wird mittels Leitung 7 die Stickstofffraktion zur Druckwechseladsorption B zurückgeführt und in dieser als Regeneriergas für die mit Kohlendioxid beladenen Adsorber verwendet.

**Figur 2** zeigt eine Kombination aus einer Amin-Wäsche D zur Kohlendioxidentfernung, einer Adsorptionsanlage E zur Trocknung des Einsatzgasgemisches und einer kryogenen Destillation A zur Abtrennung der $C_{2+}$-Kohlenwasserstoffe. Das in der Amin-Wäsche D abgetrennte Kohlendioxid wird über Leitung 8, das in der Adsorptionsanlage E gewonnene Wasser über Leitung 6 und die in der kryogenen Destillation A abgetrennten $C_{2+}$-Fraktion über Leitung 5 abgeführt.

**Figur 3** zeigt das erfindungsgemäße Verfahren, bestehend aus einer Adsorptionsanlage E zur Trocknung des Einsatzgasgemisches, einer Membrantrennanlage F zur Abtrennung von Kohlendioxid sowie einer Tieftemperatur-Destillation A zur Zerlegung des getrockneten und von Kohlendioxid teilweise befreiten Gasstromes in eine flüssige Methanproduktfraktion, die über Leitung 4 abgegeben wird, sowie in eine $C_{2+}$- und $CO_2$-enthaltende Fraktion, die über Leitung 5 abgegeben wird. Die aus der Membrantrennanlage F über Leitung 9 abgezogene Kohlendioxidfraktion kann zusammen mit der über Leitung 5 aus der Tieftemperatur-Destillation A abgezogenen $C_{2+}$- und $CO_2$-reichen Fraktion und einem über Leitung 10 herangeführten Boil-off-Gas, das aus einem diesem Verfahren nachgeschalteten Methantank stammt, über Leitung 11 der Adsorptionsanlage E als Regeneriergas zum Regenerieren der mit Wasser beladenen Adsorber zugeführt werden.

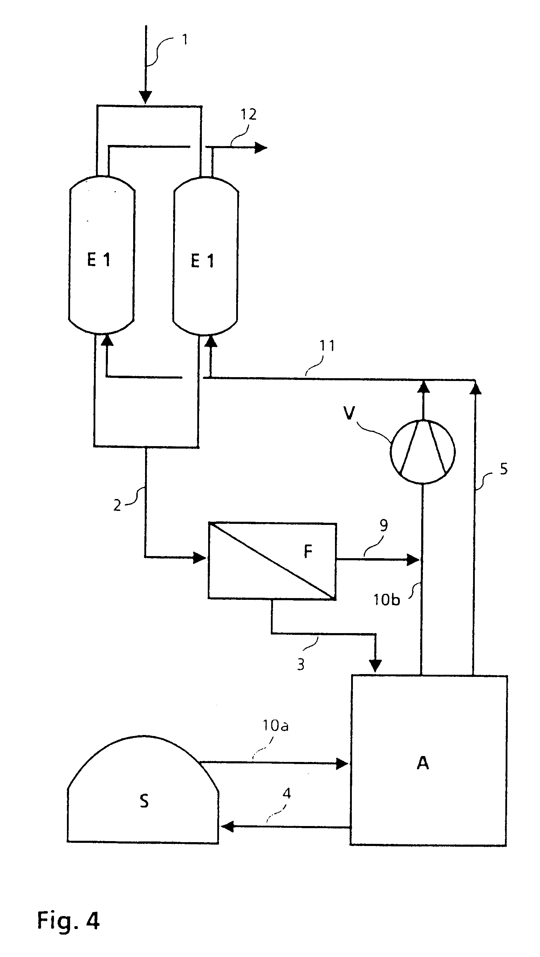

**Figur 4** zeigt ein zur vorherigen Figur 3 analoges Ausführungsbeispiel des erfindungsgemäßen Verfahrens. Die Trocknung des Einsatzgasgemisches aus Leitung 1 erfolgt hierbei in einer aus mindestens zwei Adsorbern E1 und E2 bestehenden Adsorptionsanlage. Das getrocknete Einsatzgasgemisch wird über Leitung 2 einer Membrantrennanlage F zugeführt, in dieser das Kohlendioxid abgetrennt und über Leitung 9 aus der Membrantrennanlage F geführt. Das nunmehr im wesentlichen aus Methan, $C_{2+}$-Kohlenwasserstoffen und Stickstoff bestehende Gasgemisch wird mittels Leitung 3 einer Tieftemperatur-Anlage A zugeführt, in dieser die $C_{2+}$-Kohlenwasserstoffe sowie das restliche Kohlendioxid durch Destillation und der Stickstoff durch Entspannen abgetrennt und über Leitung 5 aus der Tieftemperatur-Anlage A geführt. Der bei der Tieftemperatur-Destillation gewonnene hochreine flüssige Methanproduktstrom wird mittels Leitung 4 in den Flüssigmethan-Speichertank S befördert. Das aus dem Flüssigmethan-Speichertank S entweichende Boil-off-Gas wird über Leitung 10a in die Tieftemperatur-Anlage A zurückgeführt, in dieser zur Kältebereitstellung angewärmt und anschließend über Leitung 10b, nach zuvor erfolgter Beimischung des $CO_2$-reichen Verfahrensstromes aus der Membrantrennanlage in Leitung 9, Verdichter V zugeführt. Nach der Verdichtung wird dieser Verfahrensstrom mit der $C_2$-reichen Fraktion in Leitung 5 vermischt und den Adsorbern E1 und E2 über Leitung 11 als Regeneriergas zugeführt. Das mit Wasser beladene Regeneriergas verläßt die Anlage über Leitung 12.

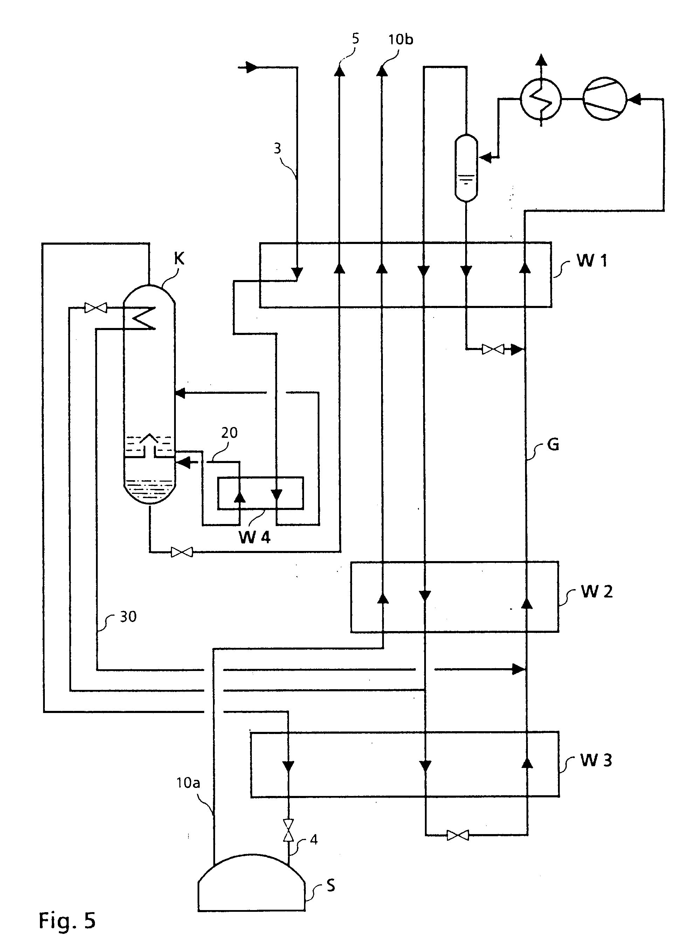

**Figur 5** zeigt ein Ausführungsbeispiel für den Tieftemperaturverfahrensschritt. Zur Bereitstellung der benötigten Prozeßkälte findet ein Kältemittelgemischkreislauf G Verwendung. Der aus der Membrantrennanlage F (Fig. 3) über Leitung 3 in die Tieftemperatur-Destillation geführte Verfahrensstrom wird zunächst im Gegenstrom zu anzuwärmenden Verfahrensströmen im Wärmetauscher W1 abgekühlt. Vor seiner Einspeisung in die Kolonne K erwärmt er im Wärmetauscher W4 einen aus der Kolonne K über Leitung 20 abgezogenen $C_2$-reichen Strom, der darauf unterhalb der Abzugsstelle wieder in die Kolonne K zurückgeführt wird. Der Kopf der Kolonne K wird mittels eines in Leitung 30 aus dem Kältemittelgemischkreislauf G abgezogenen Teilstromes gekühlt. Am Sumpf der Kolonne K wird über Leitung 5 eine

3

$C_{2+}$-Kohlenwasserstoff-Fraktion, in der das restliche Kohlendioxid enthalten ist, abgezogen, im Wärmetauscher W1 angewärmt und anschließend aus der Tieftemperatur-Anlage geführt. Am Kopf der Kolonne K wird eine hochreine Methanfraktion gewonnen, im Wärmetauscher W3 verflüssigt und über Leitung 4 in den Flüssigmethan-Speichertank S geführt. Das aus diesem Flüssigmethan-Speichertank S austretende Boil-off-Gas wird über Leitung 10a zurück in die Tieftemperatur-Anlage geführt, in dieser in den Wärmetauschern W2 und W1 erwärmt und danach über Leitung 10b aus der Tieftemperatur-Anlage abgeführt.

Aus **Tabelle 1** ist eine Materialbilanz ersichtlich, wie sie sich bei der Durchführung des in den Figuren 4 und 5 dargestellten Verfahrens ergibt. Hierbei sind die aus der Materialbilanz ersichtlichen Daten an den Verfahrenspunkten berechnet, zu denen die jeweiligen Bezugszeichenpfeile weisen.

Tabelle 1

| Leitung | 1 | 2 | 3 | 4 | S | 10a | 10b | 5 | 9 | 11 |
|---|---|---|---|---|---|---|---|---|---|---|
| $N_2$ [Mol-%] | 0,75 | 0,75 | 0,77 | 0,88 | 0,31 | 8,13 | 8,13 | 0,00 | 0,53 | 1,99 |
| $CH_4$ [Mol-%] | 88,75 | 88,77 | 90,28 | 99,12 | 99,69 | 91,87 | 91,87 | 29,97 | 71,85 | 57,25 |
| $C_{2+}$ [Mol-%] | 6,56 | 6,55 | 6,95 | 0,00 | 0,00 | 0,00 | 0,00 | 54,41 | 2,25 | 25,48 |
| $CO_2$ [Mol-%] | 3,92 | 3,93 | 2,00 | 0,00 | 0,00 | 0,00 | 0,00 | 15,62 | 25,37 | 15,22 |
| $H_2O$ [Mol-%] | 0,02 | 0,00 | 0,00 | 0,00 | 0,00 | 0,00 | 0,00 | 0,00 | 0,00 | 0,06 |
| T [K] | 300,0 | 300,0 | 300,0 | 119,3 | 111,7 | 144,3 | 305,0 | 305,0 | 300,0 | 307,0 |
| p [bar] | 35,0 | 34,0 | 33,5 | 32,1 | 1,1 | 1,1 | 1,1 | 13,0 | 1,1 | 11,4 |

## Patentansprüche

1. Verfahren zur Gewinnung von hochreinem flüssigen Methan aus einem im wesentlichen aus Methan, $C_{2+}$-Kohlenwasserstoffen, Kohlendioxid und Stickstoff bestehenden Einsatzgasgemisch, **dadurch ge-**

**kennzeichnet,** daß

a) das Einsatzgasgemisch (1) zunächst einer Adsorptionsanlage (E) zugeführt und in dieser von Wasser befreit,

b) das getrocknete Einsatzgasgemisch (2) einer Membrantrennanlage (F) zugeführt und in dieser das Kohlendioxid bis auf einen Restgehalt kleiner als 2 Vol-% abgetrennt (9) und

c) das nunmehr im wesentlichen aus Methan, $C_{2+}$-Kohlenwasserstoffen und Stickstoff bestehende Gasgemisch (3) einer Tieftemperatur-Destillation (A) zugeführt und in dieser die $C_{2+}$-Kohlenwasserstoffe sowie der Restgehalt an Kohlendioxid durch Destillation und der Stickstoff durch Entspannen abgetrennt werden (5), wodurch ein hochreiner flüssiger Methanproduktstrom (4) gewonnen wird.

2. Verfahren nach Anspruch 1, dadurch gekennzeichnet, daß die in der Membrantrennanlage gewonnene Kohlendioxidfraktion zusammen mit der $C_{2+}$-Kohlenwasserstofffraktion aus der Tieftemperatur-Destillation und dem Boil-off-Gas aus einem diesem Verfahren nachgeschaltetem Flüssigmethan-Speichertank vermischt und der Adsorptionsanlage als Regeneriergas zugeführt wird.

3. Verfahren nach Anspruch 2, dadurch gekennzeichnet, daß die in der Membrantrennanlage gewonnene Kohlendioxidfraktion zusammen mit dem Boil-off-Gas vor der Vermischung mit der $C_{2+}$-Kohlenwasserstofffraktion ein- oder mehrstufig verdichtet wird.

4. Verfahren nach einem der Ansprüche 1 bis 3, dadurch gekennzeichnet, daß die Membrantrennanlage zweistufig ausgeführt ist.

5. Verfahren nach einem der Ansprüche 1 bis 4, dadurch gekennzeichnet, daß in der Tieftemperatur-Destillation eine Kolonne zur Abtrennung der $C_{2+}$-Kohlenwasserstoffe und des restlichen Kohlendioxids verwendet wird, die mittels des im wesentlichen aus Methan, $C_{2+}$-Kohlenwasserstoffen und Stickstoff bestehenden Gasgemisches aus der Membrantrennanlage geheizt und durch einen Teilstrom des in der Tieftemperatur-Destillation verwendeten Kältekreislaufs gekühlt wird.

**Claims**

1. Process for producing highly pure liquid methane from a feed gas mixture essentially comprising methane, $C_{2+}$-hydrocarbons, carbon dioxide and nitrogen, characterized in that

a) the feed gas mixture (1) is first fed to an adsorption unit (E) and in this freed from water,

b) the dried feed gas mixture (2) is fed to a membrane separation unit (F) and in this the carbon dioxide is separated off down to a residual content less than 2% by volume (9) and

c) the gas mixture (3) now essentially comprising methane, $C_{2+}$-hydrocarbons and nitrogen is fed to a low-temperature distillation (A) and in this the $C_{2+}$-hydrocarbons and the residual content of carbon dioxide are separated off (5) by distillation and the nitrogen by pressure reduction, as a result of which a highly pure liquid methane product stream (4) is produced.

2. Process according to Claim 1, characterized in that the carbon dioxide fraction produced in the membrane separation unit is mixed together with the $C_{2+}$-hydrocarbon fraction from the low-temperature distillation and the boil-off gas from a liquid methane storage tank connected downstream of this process and is fed to the adsorption unit as regeneration gas.

3. Process according to Claim 2, characterized in that the carbon dioxide fraction produced in the membrane separation unit is compressed in a single-stage or multistage compressor together with the boil-off gas prior to mixing with the $C_{2+}$-hydrocarbon fraction.

4. Process according to any one of Claims 1 to 3, characterized in that the membrane separation unit is of two-stage design.

5. Process according to any one of Claims 1 to 4, characterized in that a column for separating off the $C_{2+}$-hydrocarbons and the residual carbon dioxide is used in the low-temperature distillation, which column is heated by the gas mixture from the membrane separation unit essentially comprising methane, $C_{2+}$-hydrocarbons and nitrogen and is cooled by a part-stream of the refrigeration circuit used in the low-temperature distillation.

**Revendications**

1. Procédé pour l'obtention de méthane liquide très pur à partir d'un mélange de gaz de départ consistant essentiellement en méthane, en hydrocarbures en $C_{2+}$, en dioxyde de carbone et en azote, caractérisé en ce que :

   a) le mélange de gaz de départ (1) est d'abord amené dans une installation d'adsorption (E) et y est débarrassé de son eau;

   b) le mélange de gaz de départ séché (2) est amené dans une installation de séparation à membrane (F) et le dioxyde de carbone est séparé (9) dans celle-ci jusqu'à une teneur résiduelle inférieure à 2% en volume, et

   c) le mélange de gaz (3) consistant à présent essentiellement en méthane, en hydrocarbures en $C_{2+}$ et en azote est amené à une distillation à basse température (A) et, dans celle-ci, les hydrocarbures en $C_{2+}$ ainsi que la teneur résiduelle en dioxyde de carbone sont séparés (5) par distillation et l'azote est séparé par détente, grâce à quoi un courant produit de méthane liquide très pur (4) est obtenu.

2. Procédé suivant la revendication 1, caractérisé en ce que la fraction de dioxyde de carbone obtenue dans l'installation de séparation à membrane est mélangée avec la fraction d'hydrocarbures en $C_{2+}$ provenant de la distillation à basse température et avec le gaz dégagé par ébullition provenant d'un réservoir de méthane liquide monté en aval de ce procédé et est amenée code gaz de régénération à l'installation d'adsorption.

3. Procédé suivant la revendication 2, caractérisé en ce que la fraction de dioxyde de carbone obtenue dans l'installation de séparation à membrane est comprimée en une ou plusieurs étapes, conjointement avec le gaz dégagé par ébullition, avant l'opération de mélange avec la fraction d'hydrocarbures en $C_{2+}$.

4. Procédé suivant l'une quelconque des revendications 1 à 3, caractérisé en ce que l'installation de séparation à membrane est réalisée avec deux étapes.

5. Procédé suivant l'une quelconque des revendications 1 à 4, caractérisé en ce que dans la distillation à basse température est utilisée une colonne pour la séparation des hydrocarbures en $C_{2+}$ et du dioxyde de carbone résiduel, qui est chauffée au moyen du mélange de gaz provenant de l'installation de séparation à membrane consistant essentiellement en méthane, en hydrocarbures en $C_{2+}$ et en azote et refroidie à l'aide d'un courant partiel du circuit de refroidissement utilisé dans la distillation à basse température.

**Fig. 1**

**Fig. 2**

**Fig. 3**

Fig. 4

Fig. 5